# FASCICULE DE BREVET EUROPEEN

(11) **EP 0 485 246 B1**
(45) Date de publication et mention de la délivrance du brevet: **14.12.1994**
(21) Numéro de dépôt: 91402667.9
(22) Date de dépôt: 07.10.1991
(51) Int. Cl.: C07C 21/18, C07C 17/00

(54) **Fabrication de fluoroéthylènes et de chlorofluoroéthylènes**
Verfahren zur Herstellung von Fluorethylenen und Chlorfluorethylenen
Process for the preparation of fluoroethylenes and chlorofluoroethylenes

(30) Priorité: 06.11.1990 FR 9013705
(43) Date de publication de la demande: 13.05.1992
(73) Titulaire: ELF ATOCHEM S.A., 92800 Puteaux (FR)
(72) Inventeur: Berthe, Bernard, F-13011 Marseille (FR); Cognion, Jean-Marie, F-69230 Saint-Genis-Laval (FR)
(74) Mandataire: Leboulenger, Jean

(56) Documents cités:
- EP-A- 0 053 657
- EP-A- 0 459 463
- US-A- 3 564 064
- US-A- 4 876 405

## Description

La présente invention concerne la préparation de fluoroéthylènes et de chlorofluoroéthylènes par hydrogénolyse catalytique de chlorofluoroéthanes ou de chlorofluoroéthylènes.

L'hydrogénolyse des chlorofluoroéthanes est une réaction connue depuis longtemps (brevet GB 698 386) et de nombreuses améliorations lui ont été apportées. On peut citer, par exemple, le brevet GB 931 643 qui utilise un catalyseur d'oxyde de chrome sur un support d'alumine pour l' hydrogénolyse du 1,1,2-trichloro-1,2,2-trifluoroéthane (F113) à des températures voisines de 500°C, et le brevet US 3 636 173 qui préconise pour l'hydrogénolyse de fluorohalocarbures un catalyseur à base de nickel ou de chrome sur un support d'alumine préalablement activé à l'acide fluorhydrique. Toutefois, les compositions catalytiques décrites dans ces brevets nécessitent des températures de fonctionnement élevées (de 300 à 600°C) et les taux de transformation ou les sélectivités sont faibles.

Plus récemment, le brevet EP 53657 décrit un procédé de deshydrochloration du F113 en chlorotrifluoroéthylène (F1113) et trifluoroéthylène (F1123) au moyen d'un catalyseur comprenant un métal du groupe du platine déposé sur un support du type fluorure de magnésium alcalin. Les résultats montrent une désactivation rapide de ce catalyseur avec une formation importante de 1,1,2-trifluoroéthane (F143) et de 1-chloro-1,2,2-trifluoroéthane (F133) comme sous-produits, et ceci même pour de faibles taux de conversion du F113.

Dans le brevet EP 355 907 est décrit un catalyseur comprenant un support poreux oxygéné imprégné avec un métal du groupe VIII et un sel d'un métal alcalin ou alcalinoterreux. Les exemples donnés montrent des taux de transformation du F113 de l'ordre de 50 % et des sélectivités en F1113 et F1123 qui ne dépassent pas 80 %.

On sait par ailleurs que les catalyseurs à base de cuivre seul sont actifs dans l'hydrogénolyse de chlorofluoroalcanes. Dans le brevet US 2 615 925 qui utilise un tel catalyseur, les températures de fonctionnement sont élevées (supérieures à 500°C) et les taux de conversion et rendements sont médiocres.

On sait aussi que les catalyseurs à base de palladium sont actifs. Cependant, le brevet US 4 876 405 montre que l'emploi d'un tel catalyseur dans l'hydrogénolyse du difluorotétrachloroéthane symétrique (F112) et du F113 conduit à une formation importante (plus de 10 %) de sous-produits saturés tels que le 1,1-difluoroéthane (F152a) et le 1,1,2-trifluoroéthane (F143).

Par ailleurs, on a déjà proposé l'emploi de catalyseurs mixtes. On peut citer le brevet US 2 864 873 relatif à des catalyseurs mixtes à base de cuivre et de nickel sur un support d'oxyde de chrome. Ces catalyseurs qui fonctionnent à des températures de 325 à 425°C conduisent à des taux de transformation du F113 en F1113 inférieurs à 50 %. On peut mentionner aussi le brevet US 2 697 124 relatif à un catalyseur mixte cuivre-cobalt sur un support de fluorure de magnésium ; malgré des températures élevées (supérieures à 400°C) pour l'hydrogénolyse du F113, ce catalyseur donne des taux de conversion du F113 inférieurs à 80 %. L'hydrogénolyse de F113 et de F114 avec des catalyseurs mixtes à base de cuivre et de chrome est également décrite dans les brevets BE 653 362 et FR 1 409 109 ; les températures de fonctionnement sont élevées, et les taux de transformation et les rendements en composés éthyléniques sont faibles. Il en est de même pour le brevet US 3 505 417 qui décrit l'hydrogénolyse du F114 en tétrafluoroéthylène sur des catalyseurs cuivre-chrome ou cuivre-cobalt.

Dans le brevet EP 459463, publié après la date de priorité et la date de dépôt de la demande, on décrit un procédé de préparation de 1-chloro-1,2,2-trifluoroéthylène ou de 1,2,2-trifluoroéthylène à partir de 1,1,2-trichloro-1,2,2-trifluoroéthane en présence d'un catalyseur contenant un métal comme le palladium, un métal comme le cuivre ou l'argent et un support comme le charbon actif; ce document ne mentionne pas explicitement un catalyseur tel que revendiqué.

Il a maintenant été trouvé que, pour l'hydrogénolyse de chlorofluoroéthanes ou de chlorofluoroéthylènes, l'emploi de catalyseurs mixtes à base de cuivre ou d'argent et d'au moins un métal du groupe du platine (ruthénium, rhodium, palladium, osmium, iridium ou platine) permet, à des températures de fonctionnement peu élevées, d'obtenir d'excellents taux de conversion et sélectivités.

L'invention a donc pour objet un procédé de fabrication de fluoroéthylènes et/ou de chlorofluoroéthylènes par hydrogénolyse catalytique d'un chlorofluoroéthane de formule générale : 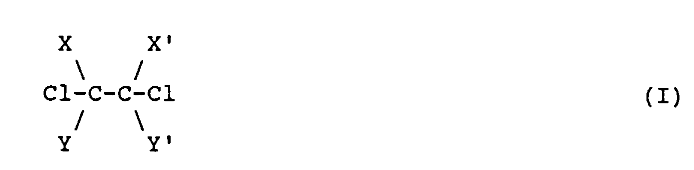
dans laquelle l'un au moins des symboles X, X', Y et Y' représente un atome de fluor et les autres, identiques ou différents, représentent chacun un atome d'hydrogène ou de chlore, ou d'un chlorofluoroéthylène de formule générale : 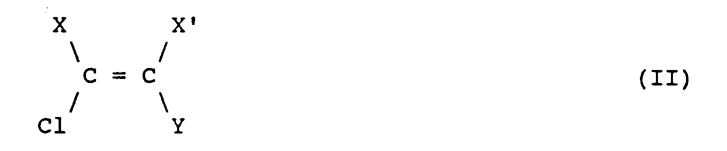
dans laquelle l'un au moins des symboles X, X' et Y représente un atome de fluor et les autres, identiques ou différents, représentent chacun un atome d'hydrogène ou de chlore, caractérisé en ce que l'on utilise un catalyseur mixte à base de cuivre ou d'argent et d'au moins un métal du groupe du platine, de préférence le palladium, ces métaux étant déposés sur un charbon actif.

Dans le catalyseur mixte selon la présente invention, la teneur en cuivre et/ou argent peut aller de 1 à 20 % en poids, de préférence entre 3 et 15 %, et celle en métal du groupe du platine peut aller de 0,1 à 10 % en poids. Le taux de transformation du chlorofluoroéthane (I) ou du chlorofluoroéthylène (II) varie dans le même sens que la teneur en métal du groupe du platine ; cependant, pour éviter la formation concomitante de fluoroéthanes, on préfère mettre en oeuvre un catalyseur contenant de 0,1 à 5 % dudit métal.

Le support de charbon actif peut se présenter sous diverses formes telles que, par exemple, extrudées, pastillées, broyées ou billes.

La préparation du catalyseur mixte selon l'invention est très simple et peut être réalisée de différentes manières, soit par co-imprégnation du support avec du cuivre ou de l'argent et au moins un métal du groupe du platine, soit par imprégnation successive des différents métaux.

L'imprégnation du support peut être effectuée, par exemple, par trempage ou pulvérisation au moyen d'une solution de sels des métaux à déposer. Cette opération peut être menée à une température allant d'environ 20 à 80°C, mais est de préférence réalisée à la température ambiante. Le solvant qui est de préférence l'eau, mais peut être aussi un hydrocarbure, un alcool léger ou une cétone, est ensuite évaporé sous vide en élevant progressivement la température. Avant utilisation, le catalyseur est réduit sous courant d'hydrogène (10 à 80 l/h pour 100 g de catalyseur) à une température comprise entre 100 et 500°C.

Comme sels métalliques pour l'imprégnation, on utilise avantageusement des chlorures. Cependant, d'autres sels (par exemple, nitrates, acétates, acétylacétonates) peuvent être mis en oeuvre ; il importe seulement que ces sels soient suffisamment solubles dans le solvant pour obtenir les concentrations pondérales en métaux désirées.

Un autre mode de préparation d'un catalyseur selon l'invention consiste à partir d'un catalyseur commercial à base du métal du groupe du platine supporté et à l'imprégner avec une solution d'un sel de cuivre ou d'argent, l'évaporation du solvant et la réduction finale étant réalisées de la même façon que dans le cas de la co-imprégnation.

Le catalyseur selon l'invention peut être mis en oeuvre suivant toute technique d'hydrogénolyse telle que, notamment, celles opérant en lit fixe ou en lit fluidisé.

Le procédé d'hydrogénolyse selon l'invention fait intervenir l'une et/ou l'autre des réactions suivantes :

XYClC-CClX'Y' + H₂ -----> XYC=CX'Y' + 2HCl

XClC=CX'Y + H₂ -----> XHC=CX'Y + HCl

Comme exemples non limitatifs de chlorofluoroéthanes (I) et de chlorofluoroéthylènes (II) qui peuvent être hydrogénolysés conformément au présent procédé, on peut mentionner plus particulièrement le 1,2-difluoro-1,1,2,2-tétrachloroéthane (F112), le 1,1,2-trichloro-1,2,2-trifluoroéthane (F113), le 1,2-dichloro-1,1,2,2-tétrafluoroéthane (F114), le 1,2-dichloro-1,1,2-trifluoroéthane (F123a), le 1,2-dichloro-1,1-difluoroéthane (F132b) et le chlorotrifluoroéthylène (F1113).

L'hydrogénolyse peut être effectuée à une température allant de 100 à 400°C, de préférence comprise entre 150 et 350°C. On opère avantageusement à la pression atmosphérique, mais on ne sortirait pas du cadre de la présente invention en travaillant à une pression sub- ou superatmosphérique.

Le rapport molaire entre l'hydrogène et le chlorofluoroéthane (I) ou chlorofluoroéthylène (II) est généralement compris entre 0,1 et 10, de préférence entre 0,5 et 5.

Le temps du contact peut varier entre 1 et 45 secondes, mais d'excellents résultats ont été obtenus avec des temps de contact compris entre 2 et 10 secondes.

Les exemples suivants illustrent l'invention sans la limiter.

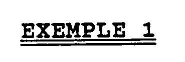

### A - PREPARATION DU CATALYSEUR

A 150 g d'un catalyseur commercial à 0,8 % de palladium sur charbon actif, sous formes d'extrudés, possédant une surface BET supérieure à 500 m²/g et ayant une surface de métal supérieure à 100 m²/g de palladium, on ajoute dans un évaporateur rotatif à 20°C 150 g d'une solution aqueuse de chlorure cuivrique contenant 15 g de cuivre. L'eau est ensuite évaporée sous vide en élevant progressivement la température jusqu'à 170°C. Le catalyseur est ensuite réduit à 170°C pendant 8 heures sous courant d'hydrogène (25 litres/heure à pression atmosphérique).

On obtient ainsi un catalyseur, prêt à l'emploi, contenant 0,7 % de palladium et 9 % de cuivre.

### B - HYDROGENOLYSE DU F113

Dans un tube en inox 316L de 4 mm de diamètre intérieur, chauffé électriquement, on introduit 1,26 ml du catalyseur décrit précédemment, puis on fait passer à pression atmosphérique un mélange d'hydrogène et de F113 aux températures, rapports molaires et débits indiqués dans le tableau suivant dont les trois dernières colonnes rassemblent les résultats obtenus.

| Température du réacteur (°C) | Rapport molaire H₂/F113 | Débit total (mole/heure) | Taux de transformation du F113 (%) | Sélectivité en : | |
|---|---|---|---|---|---|
| | | | | F1113 | F1123 |
| 250 | 3,4 | 0,045 | 98 | 81 | 16 |
| 300 | 3,4 | 0,045 | 100 | 69 | 29 |
| 320 | 5 | 0,15 | 100 | 76 | 23 |
| 320 | 4,6 | 0,045 | 100 | 55 | 42 |

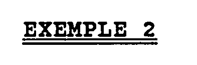

### A - PREPARATION DU CATALYSEUR

Dans un évaporateur rotatif, on charge 23 g d'un charbon actif CECA ayant une porosité de 0,6 cm³/g et une surface spécifique de 950 m²/g, sous forme d'extrudés de 1,8 mm de diamètre. On introduit 70 ml d'une solution aqueuse contenant 2,96 g de chlorure de palladium et 2,7 g de chlorure cuivrique hydraté (CuCl₂, 2H₂O), puis on évapore l'eau sous pression réduite (1 kPa) et on sèche à 100°C. On traite ensuite à 450°C pendant 3 heures sous courant d'hydrogène (10 l/h) et on obtient ainsi un catalyseur, prêt à l'emploi, contenant 7 % de palladium et 4 % de cuivre.

### B - HYDROGENOLYSE DU F113

En opérant comme à l'exemple 1B mais avec 1,26 ml de ce catalyseur à 7 % de palladium et 4 % de cuivre, on a obtenu les résultats rassemblés dans le tableau suivant :

| Température du réacteur (°C) | Rapport molaire H₂/F113 | Débit total (mole/heure) | Taux de transformation du F113 (%) | Sélectivité en : | |
|---|---|---|---|---|---|
| | | | | F1113 | F1123 |
| 150 | 4 | 0,03 | 85 | 60 | 20 |
| 200 | 4 | 0,033 | 100 | 75 | 20 |
| 200 | 3 | 0,04 | 100 | 85 | 10 |
| 200 | 5 | 0,134 | 98 | 65 | 26 |

Dans cette série d'essais, le sous-produit principal est le F123a (CF₂Cl-CFClH) qui peut être recyclé au réacteur pour former du F1123.

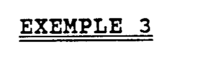

On opère comme à l'exemple 1B pour hydrogénolyser du F1113 avec 1,26 ml du catalyseur de l'exemple 2A.

Le tableau suivant rassemble les conditions opératoires de ces essais et les résultats obtenus.

| Température du réacteur (°C) | Rapport molaire H₂/F1113 | Débit total (mole/heure) | Taux de transformation du F1113 (%) | Sélectivité en F1123 (%) |
|---|---|---|---|---|
| 250 | 0,8 | 0,045 | 15 | 98 |
| 300 | 0,8 | 0,045 | 40 | 96 |

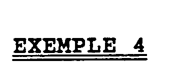

On opère comme à l'exemple 1B pour l'hydrogénolyse du F132b (CF₂Cl-CH₂Cl) avec 1,26 ml du catalyseur de l'exemple 1A.

Les conditions opératoires et les résultats obtenus sont rassemblés dans le tableau suivant.

| Température du réacteur (°C) | Rapport molaire H₂/F132b | Débit total (mole/heure) | Taux de transformation du F132b (%) | Sélectivité en CF₂ = CH₂ (%) |
|---|---|---|---|---|
| 250 | 5 | 0,056 | 20 | 99 |
| 270 | 5 | 0,056 | 46 | 99 |
| 300 | 5 | 0,056 | 76 | 98 |

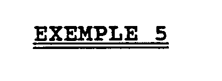

Dans un tube en Inconel de 45 cm de long et de 2,72 cm de diamètre intérieur, chauffé électriquement, on introduit 50 ml du catalyseur décrit à l'exemple 1A, puis on y fait passer un mélange d'hydrogène et de F114 aux rapports molaires, débits et températures indiqués dans le tableau suivant dont la dernière partie rassemble les résultats obtenus.

| Température du réacteur (°C) | Rapport molaire H₂/F114 | Débit total (mole/heure) | Taux de transformation du F114 (%) | Sélectivité en C₂F₄ (%) |
|---|---|---|---|---|
| 250 | 4 | 0,25 | 47 | - |
| 280 | 4 | 0,25 | 70 | - |
| 300 | 4 | 0,25 | 100 | 80 |
| 300 | 2 | 0,15 | 100 | 80 |

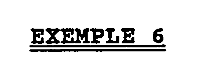

Dans le même réacteur qu'à l'exemple 5, on introduit 25 ml (13,5 g) du catalyseur décrit à l'exemple 1A et procède sous pression atmosphérique à l'hydrogénolyse du F113.

Les conditions opératoires et les résultats obtenus sont rassemblés dans le tableau suivant.

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 177 | 1 | 0,7 | 21,5 | 94,3 | 5,6 |
| 177 | 3 | 0,7 | 33 | 91,4 | 8,6 |
| 177 | 5 | 0,7 | 46 | 89,6 | 10,4 |
| 235 | 3 | 0,7 | 92 | 89,7 | 9,7 |
| 289 | 3 | 0,7 | 99 | 84,6 | 14,2 |
| 395 | 3 | 0,7 | 99,7 | 70,2 | 29,6 |

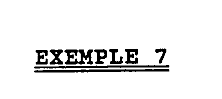

On répète l'exemple 6 avec 25 ml (13,5 g) du catalyseur décrit à l'exemple 2A. Le tableau suivant rassemble les conditions opératoires et les résultats obtenus.

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 350 | 3 | 0,72 | 100 | 60,5 | 39,2 |
| 350 | 3,1 | 0,31 | 100 | 50,5 | 48,2 |
| 350 | 3 | 0,15 | 100 | 41 | 57 |
| 350 | 3 | 0,08 | 100 | 39,1 | 58,3 |

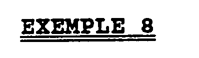

### A - PREPARATION DU CATALYSEUR

Dans un évaporateur rotatif, on charge 27 g d'un charbon actif CECA ayant une porosité de 0,87 cm³/g et une surface spécifique de 1140 m²/g, sous forme d'extrudés de 3 mm de diamètre. On introduit 100 ml d'une solution aqueuse contenant 3,5 g de chlorure de palladium et 1,62 g de chlorure cuivrique hydraté (CuCl₂, 2H₂O), puis on évapore l'eau sous pression réduite (1kPa) et on sèche à 100°C. On traite ensuite à 450°C pendant 3 heures sous courant d'hydrogène (10 l/h) et on obtient ainsi un catalyseur, prêt à l'emploi, contenant 7 % de palladium et 2 % de cuivre.

### B - HYDROGENOLYSE DU F113

En opérant dans le même réacteur qu'à l'exemple 5, mais avec 25 ml de ce catalyseur à 7 % de palladium et 2 % de cuivre, on a obtenu les résultats rassemblés dans le tableau suivant :

| Température du réacteur (°C) | Rapport molaire H₂/F113 | Débit total (mole/hheure) | Taux de transformation du F113 (%) | Sélectivité en | |
|---|---|---|---|---|---|
| | | | | F1113 (%) | F1123 (%) |
| 202 | 3 | 0,33 | 100 | 49,6 | 38 |
| 146 | 3 | 0,32 | 43 | 56,1 | 30 |
| 171 | 3 | 0,33 | 81 | 55,5 | 32 |
| 235 | 3 | 0,33 | 100 | 47,4 | 43,4 |
| 146 | 2 | 0,72 | 19 | 55 | 32,2 |
| 171 | 2 | 0,71 | 48 | 57,7 | 33 |
| 202 | 2 | 0,71 | 78,8 | 61 | 30,3 |
| 225 | 2 | 0,72 | 91 | 57,3 | 34,7 |
| 273 | 2 | 0,72 | 94 | 55,6 | 38,2 |
| 247 | 3 | 0,71 | 99,2 | 56 | 37,4 |
| 204 | 3 | 0,71 | 79 | 56 | 35,6 |
| 346 | 3 | 0,71 | 100 | 52,7 | 45,2 |
| 401 | 3 | 0,71 | 100 | 51,6 | 48,3 |

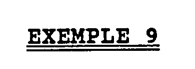

On opère comme à l'exemple 8A, mais en remplaçant la solution aqueuse de chlorure de palladium et de chlorure cuivrique par 100 ml d'une solution pyridinique contenant 4,17 g d'acétate d'argent et 0,506 g d'acétate de palladium.

On obtient ainsi un catalyseur, prêt à l'emploi, contenant 0,8 % de palladium et 9 % d'argent. En opérant dans le même réacteur qu'à l'exemple 5 avec 25 ml de ce catalyseur, on a procédé à l'hydrogénolyse du F113 à pression atmosphérique et obtenu les résultats rassemblés dans le tableau suivant.

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 151 | 3,1 | 0,71 | 1,6 | 88 | 2,4 |
| 191 | 3 | 0,72 | 12,2 | 91 | 1 |
| 234 | 3 | 0,71 | 47,6 | 94 | 1,3 |

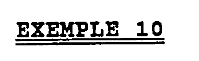

On opère comme à l'exemple 8A, mais en remplaçant la solution aqueuse de chlorure de palladium et de chlorure cuivrique par 100 ml d'une solution aqueuse contenant 0,5 g de chlorure de ruthénium hydraté (teneur en Ru : 41,5 %) et 7,3 g de chlorure cuivrique hydraté (CuCl₂, 2H₂O).

On obtient ainsi un catalyseur, prêt à l'emploi, contenant 0,7 % de ruthénium et 9 % de cuivre. En utilisant 25 ml de ce catalyseur pour hydrogénolyser du F113 à pression atmosphérique dans le même réacteur qu'à l'exemple 5, on a obtenu les résultats rassemblés dans le tableau suivant.

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 180 | 3 | 0,7 | 11 | 90 | 9,7 |
| 235 | 3 | 0,7 | 65,5 | 86,5 | 8,3 |
| 259 | 3 | 0,7 | 91 | 88,8 | 10,8 |
| 278 | 3 | 0,7 | 97,5 | 86,2 | 13,3 |

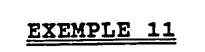

On opère comme à l'exemple 8A, mais en remplaçant la solution aqueuse de chlorure de palladium et de chlorure cuivrique par 100 ml d'une solution aqueuse contenant 0,65 g d'acide chloroplatinique hexahydraté (H₂PtCl₆, 6H₂O) et 7,25 g de chlorure cuivrique hydraté (CuCl₂, 2H₂O).

On obtient ainsi un catalyseur, prêt à l'emploi, contenant 0,8 % de platine et 9 % de cuivre. En utilisant 25 ml de ce catalyseur pour hydrogénolyser le F113 à pression atmosphérique dans le même réacteur qu'à l'exemple 5, on a obtenu les résultats rassemblés dans le tableau suivant.

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 180 | 3 | 0,18 | 3 | 98 | 2 |
| 235 | 3 | 0,18 | 23,2 | 98,3 | 1,7 |
| 290 | 3 | 0,17 | 80 | 96,2 | 3 |
| 320 | 3 | 0,18 | 72 | 93 | 5,5 |
| 235 | 3 | 0,08 | 41 | 98 | 1,2 |
| 290 | 3 | 0,08 | 99 | 94 | 4 |
| 260 | 3 | 0,08 | 75 | 97 | 2 |

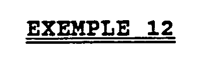

### A - PREPARATION DU CATALYSEUR

Dans un évaporateur rotatif, on charge 26 g d'un charbon actif CECA ayant une porosité de 0,22 cm³/g et une surface spécifique de 1000 m²/g, sous forme de cassons de 2 à 2,5 mm. On introduit 100 ml d'une solution aqueuse contenant 0,61 g de chlorure de rhodium RhCl₃ hydraté (40,4 % de rhodium) et 7,29 g de chlorure cuivrique hydraté (CuCl₂, 2H₂O), puis on évapore l'eau sous pression réduite (1kPa) et on sèche à 100°C. On traite ensuite à 450°C pendant 3 heures sous courant d'hydrogène (10 l/h) et on obtient ainsi un catalyseur, prêt à l'emploi, contenant 0,85 % de rhodium et 9,4 % de cuivre.

### B - HYDROGENOLYSE DU F113

En opérant dans le même réacteur qu'à l'exemple 5, mais avec 25 ml de ce catalyseur à 0,85 % de rhodium et 9,4 % de cuivre, on a obtenu les résultats rassemblés dans le tableau suivant :

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 180 | 3 | 0,17 | 4 | 70,5 | 29,5 |
| 235 | 3 | 0,17 | 33 | 74 | 24 |
| 290 | 3 | 0,17 | 91,3 | 76,3 | 23 |

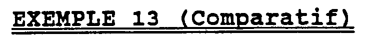

### A - PREPARATION DU CATALYSEUR

Dans un évaporateur rotatif, on charge 27 g d'un charbon actif CECA ayant une porosité de 0,87 cm³/g et une surface spécifique de 1140 m²/g, sous forme d'extrudés de 3 mm de diamètre. On introduit 100 ml d'une solution aqueuse contenant 7,3 g de chlorure cuivrique hydraté (CuCl₂, 2H₂O), puis on évapore l'eau sous pression réduite (1kPa) et on sèche à 100°C. On traite ensuite à 450°C pendant 3 heures sous courant d'hydrogène (10 l/h) et on obtient ainsi un catalyseur, prêt à l'emploi, contenant 9 % de cuivre.

### B - HYDROGENOLYSE DU F113

En opérant dans le même réacteur qu'à l'exemple 5, mais avec 25 ml de ce catalyseur contenant 9 % de cuivre, on a obtenu les résultats rassemblés dans le tableau suivant :

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 171 | 3 | 0,72 | 0,14 | 79,4 | 20,6 |
| 230 | 3 | 0,71 | 0,95 | 84 | 11,6 |
| 288 | 3 | 0,70 | 3,30 | 97 | 1,4 |
| 340 | 3 | 0,72 | 15,40 | 98 | 1 |

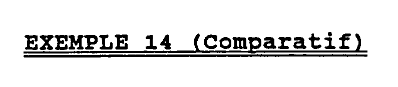

### A - PREPARATION DU CATALYSEUR

Dans un évaporateur rotatif, on charge 23 g d'un charbon actif CECA ayant une porosité de 0,60 cm³/g et une surface spécifique de 947 m²/g, sous forme d'extrudés de 1,8 mm de diamètre. On introduit 50 ml d'une solution aqueuse contenant 0,39 g de chlorure de palladium, puis on évapore l'eau sous pression réduite (1kPa) et on sèche à 100°C. On traite ensuite à 400°C pendant 2 heures sous courant d'hydrogène (6 l/h) et on obtient ainsi un catalyseur, prêt à l'emploi, contenant 1 % de palladium.

### B - HYDROGENOLYSE DU F113

En opérant dans le même réacteur qu'à l'exemple 5, mais avec 25 ml de ce catalyseur à 1 % de palladium, on a obtenu les résultats rassemblés dans le tableau suivant :

| Température du réacteur (°C) | Rapport molaire H₂/F113 | Débit total (mole/heure) | Taux de transformation du F113 (%) | Sélectivité en | |
|---|---|---|---|---|---|
| | | | | F1113 (%) | F1123 (%) |
| 118 | 3 | 0,70 | 2,8 | 5,5 | 43,8 |
| 223 | 3 | 0,70 | 78,6 | 5,9 | 59 |
| 245 | 3 | 0,68 | 93,6 | 3,8 | 64,8 |

## Revendications

1. Procédé de fabrication de fluoroéthylènes et/ou de chlorofluoroéthylènes par hydrogénolyse catalytique d'un chlorofluoroéthane de formule générale : 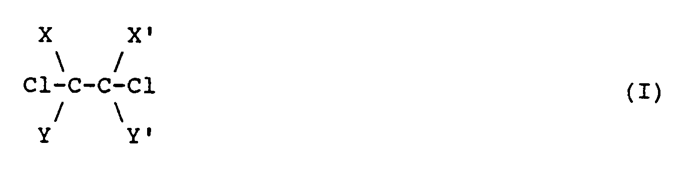 dans laquelle l'un au moins des symboles X, X', Y et Y' represente un atome de fluor et les autres, identiques ou différents, représentent chacun un atome d'hydrogène ou de chlore, ou d'un chlorofluoroéthylène de formule générale : 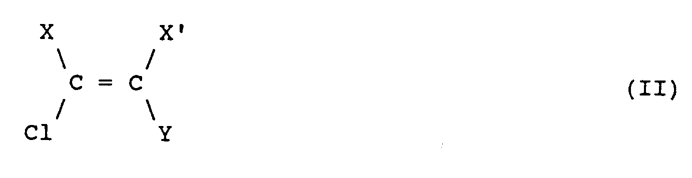 dans laquelle l'un au moins des symboles X, X' et Y représente un atome de fluor et les autres, identiques ou différents, représentent chacun un atome d'hydrogène ou de chlore, caractérisé en ce que l'on utilise un catalyseur mixte à base de cuivre ou d'argent et d'au moins un métal du groupe du platine, ces métaux étant déposés sur un charbon actif.

2. Procédé selon la revendication 1, dans lequel la teneur en cuivre et/ou argent du catalyseur est comprise entre 1 et 20 %, de préférence entre 3 et 15 %, et celle en métal du groupe du platine est comprise entre 0,1 et 10 %.

3. Procédé selon la revendication 2, dans lequel la teneur en métal du groupe du platine est comprise entre 0,1 et 5 %.

4. Procédé selon l'une des revendications 1 à 3, dans lequel le catalyseur est à base de cuivre et de palladium.

5. Procédé selon l'une des revendications 1 à 4, dans lequel on effectue l'hydrogénolyse en lit fixe ou en lit fluidisé.

6. Procédé selon l'une des revendications 1 à 5, dans lequel on opère à une température allant de 100 à 400°C, de préférence entre 150 et 350°C.

7. Procédé selon l'une des revendications 1 à 6, dans lequel on opère à pression atmosphérique.

8. Procédé selon l'une des revendications 1 à 7, dans lequel le rapport molaire entre l'hydrogène et le composé (I) ou (II) est compris entre 0,1 et 10, de préférence entre 0,5 et 5.

9. Procédé selon l'une des revendications 1 à 8, dans lequel le temps de contact est compris entre 1 et 45 secondes, de préférence entre 2 et 10 secondes.

## Claims

1. Process for the manufacture of fluoroethylenes and/or chlorofluoroethylenes by catalytic hydrogenolysis of a chlorofluoroethane of general formula: 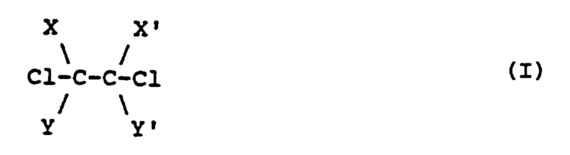 in which at least one of the symbols X, X', Y and Y' represents a fluorine atom and the others, which may be identical or different, each represent a hydrogen or chlorine atom, or of a chlorofluoroethylene of general formula: 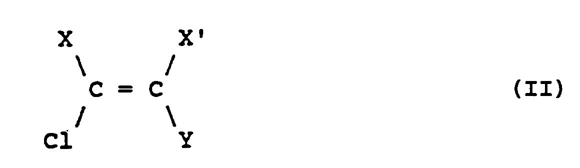 in which at least one of the symbols X, X' and Y represents a fluorine atom and the others, which may be identical or different, each represent a hydrogen or chlorine atom, characterised in that a mixed catalyst based on copper or silver and on at least one platinum group metal is used, these metals being deposited on an active charcoal.

2. Process according to Claim 1, in which the copper and/or silver content in the catalyst is between 1 and 20 %, and preferably between 3 and 15 %, and that of the platinum group metal is between 0.1 and 10 %.

3. Process according to Claim 2, in which the content of the platinum group metal is between 0.1 and 5 %.

4. Process according to one of Claims 1 to 3, in which the catalyst is based on copper and on palladium.

5. Process according to one of Claims 1 to 4, in which the hydrogenolysis is performed employing fixed-bed or fluidised-bed operation.

6. Process according to one of Claims 1 to 5, in which the reaction is performed at a temperature ranging from 100 to 400°C, and preferably between 150 and 350°C.

7. Process according to one of Claims 1 to 6, in which the reaction is performed at atmospheric pressure.

8. Process according to one of Claims 1 to 7, in which the mole ratio of hydrogen to the compound (I) or (II) is between 0.1 and 10, and preferably between 0.5 and 5.

9. Process according to one of Claims 1 to 8, in which the contact time is between 1 and 45 seconds, and preferably between 2 and 10 seconds.

## Patentansprüche

1. Verfahren zur Herstellung von Fluorethylenen und/oder Chlorfluorethylenen durch katalytische Hydrogenolyse eines Chlorfluorethans der allgemeinen Formel 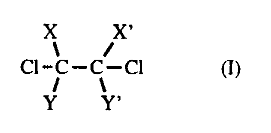 in der mindestens eines der Symbole X, X', Y und Y' ein Fluoratom darstellt und die anderen, gleich oder verschieden, jeweils ein Wasserstoff- oder Chloratom darstellen,
oder eines Chlorfluorethylens der allgemeinen Formel 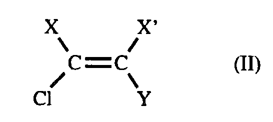 in der mindestens eines der Symbole X, X' und Y ein Fluoratom darstellt und die anderen gleich oder verschieden, jeweils ein Wasserstoff- oder ein Chloratom darstellen,
dadurch gekennzeichnet, daß man einen Mischkatalysator auf Basis von Kupfer oder Silber und mindestens eines Metalls der Platingruppe verwendet; diese Metalle werden auf Aktivkollle aufgebracht.

2. Verfahren nach Anspruch 1, wobei der Gehalt des Katalysators an Kupfer und/oder Silber zwischen 1 und 20 %, vorzugsweise zwischen 3 und 15 %, und der an Metall aus der Platingruppe zwischen 0,1 und 10 % liegt.

3. Verfahren nach Anspruch 2, wobei der Gehalt an Metall der Platingruppe zwischen 0,1 und 5 % liegt.

4. Verfahren nach einem der Ansprüche 1 bis 3, wobei der Katalysator auf Basis von Kupfer und Palladium ist.

5. Verfahren nach einem der Ansprüche 1 bis 4, wobei man die Hydrogenolyse in einem Festbett oder einem Fließbett durchführt.

6. Verfahren nach einem der Ansprüche 1 bis 5, wobei man bei einer Temperatur von 100 bis 400 °C, vorzugsweise zwischen 150 und 350 °C verfährt.

7. Verfahren nach einem der Ansprüche 1 bis 6, wobei man unter Atmosphärendruck verfährt.

8. Verfahren nach einem der Ansprüche 1 bis 7, wobei das molare Verhältnis zwischen Wasserstoff und der Verbindung (I) oder (II) zwischen 0,1 und 10, vorzugsweise zwischen 0,5 und 5, liegt.

9. Verfahren nach einem der Ansprüche 1 bis 8, wobei die Kontaktzeit zwischen 1 und 45 Sekunden, vorzugsweise zwischen 2 und 10 Sekunden, liegt.
